# EUROPÄISCHE PATENTANMELDUNG

(11) **EP 2 735 301 A1**
(43) Veröffentlichungstag der Anmeldung: **28.05.2014**
(21) Anmeldenummer: 13189203.6
(22) Anmeldetag: 18.10.2013
(51) Int. Cl.: A61K 8/37, A61K 8/34, A61Q 19/00, A61Q 15/00, A61Q 19/10, A61K 8/06, C12F 5/00

(54) **Verwendung von Triethylcitrat als Vergällungsmittel für Ethanol**

(30) Priorität: 23.11.2012 DE 102012221460
(71) Anmelder: Beiersdorf AG, 20253 Hamburg (DE)
(72) Erfinder: Triller, Benjamin, 22941 Jersbek (DE); Hüttl, Matthias, 50939 Köln (DE)

(57) **Zusammenfassung**

Verwendung von Triethylcitrat als Vergällungsmittel für Ethanol.

## Beschreibung

Die Erfindung beschreibt ein Vergällungsmittel für Ethanol, welches fermentativ und damit Naturkosmetik-konform gewonnen werden kann und sich in verschiedensten kosmetischen Formulierungen einsetzen lässt, wobei der Geruch und die Farbe der Produkte nicht merklich beeinflusst wird und eine gute Hautverträglichkeit gewährleistet ist.

Ethanol ist im Bereich der Kosmetik ein weit verbreiteter Inhaltsstoff. Dieser dient u.a. als Lösungsmittel oder auch zur Unterstützung der Konservierung eines Kosmetikproduktes. Zur Konservierungsunterstützung wird Ethanol besonders in dem Bereich der sogenannten Naturkosmetik verwendet, da die allgemein, anerkannten Naturkosmetik-Standards die Verwendung von Konservierungsmittel stark einschränken.

Ethanol unterliegt der Branntweinsteuer, sofern der Einsatz nicht zur Herstellung von Arzneimitteln oder Lebensmittel wie Essig erfolgt. Um diese Steuer zu vermeiden, werden Vergällungsmittel dem Ethanol zugesetzt mit dem Ziel, dass der Ethanol ungenießbar wird. An Vergällungsmittel im Bereich der Kosmetik werden besonders hohe Anforderungen gestellt, da hier für die Verwendung weder gesundheitliche Bedenken bestehen dürfen, noch der Geruch oder die Farbe das Produkt beeinflussen darf. Hinzu kommt die Formulierbarkeit in verschiedenen kosmetischen Produkten wie z.B. Wasser in Öl- oder Öl in Wasser-Emulsion, wässrigen Gelen oder Wässer, die gewährleistet sein muss.

Als Typische Vergällungsmittel im kosmetischen Bereich sind Bitrex ®, Isopropanol, tert.-Butylalkohol, Diethylphthalat, Thymol oder Moschusketon im Einsatz, wobei bei teilweise gesundheitliche Bedenken bestehen. [1] Generell besteht in einigen Ländern der EU wie z.B. Deutschland die Möglichkeit weitere Stoffe oder Stoffgemische als Sondervergällungsmittel für den Einsatz in kosmetischen Produkten zuzulassen, sofern der Ethanol dadurch ungenießbar wird und eine Trennung der Stoffe nicht wirtschaftlich sinnvoll ist.

Für den Bereich der Naturkosmetik gelten weitere Einschränkungen. Eine gesetzliche Definition des Begriffs "Naturkosmetik" gibt es allerdings weder national noch europaweit. Es gibt jedoch formelle Kriterien, welche Anforderungen an derartige Produkte zu stellen sind. Naturkosmetik-Produkte setzen sich überwiegend aus Naturstoffen, die lediglich mit Hilfe physikalischer Prozesse gewonnen werden, und aus chemische veränderten Naturstoffen zusammen, deren chemische Reaktion limitiert sind und sich an physiologische Prozesse als Vorbild orientieren. An die auf diese Weise gewonnenen Inhaltsstoffe wird darüber hinaus noch die Anforderung gestellt, dass diese frei von gentechnisch veränderten Organismen sind und eine leichte biologische Abbaubarkeit gegeben ist.

Aufgrund des Mangels an einem geeigneten Vergällungsmittel für Ethanol sind nach einigen Standards wie ECOCERT- und COSMOS-Standard bestimmte Vergällungsmittel als Ausnahme von den Kriterien zugelassen, die den üblichen Naturkosmetik-Kriterien nicht entsprechen.

Allgemein zugelassene Vergällungsmittel für Kosmetik sind z.B. für Deutschland im §50 (4) der Verordnung zur Durchführung des Branntweinmonopolgesetzes aufgeführt:

"Zur Vergällung von 100 Liter Alkohol werden folgende Vergällungsmittel zugelassen: zur Herstellung von kosmetischen Mitteln oder Mitteln zur Geruchsverbesserung:
a) 0,5 Kilogramm Phthalsäurediethylester,
b) 0,5 Kilogramm Thymol,
c) 5,0 Kilogramm Isopropanol und 78,0 Gramm Tertiärbutanol,
d) 0,8 Gramm Denatoniumbenzoat und 78,0 Gramm Tertiärbutanol;" [7]

Das Vergällungsmittel Thymol lässt sich zwar gemäß den üblichen Naturkosmetik-Kriterien herstellen, weist jedoch eine schlechte Wasserlöslichkeit auf und ist hinsichtlich der Auswirkung auf die Gesundheit in den erforderlichen Konzentrationen als bedenklich zu bewerten. So wird bei Thymol wegen seiner Strukturähnlichkeiten zu Cresolen mit einer analogen Wirkweise in Verbindung gebracht und nur in geringen Konzentrationen als sicher zur Verwendung in kosmetischen Produkten bewertet. [6]

Weiterhin ist auch die Verwendung von Parfümölen als Sondervergällungsmittel bekannt. Dadurch lassen sich keine parfümfreier Produkte herstellen und die Duftrichtung wäre durch das Vergällungsmittel vorbestimmt.

Daher ist ein flexibleres Vergällungsmittel für verschiedenste kosmetische Produktformen wünschenswert, welches nicht in den eingesetzten Konzentrationen gesundheitlich bedenklich ist, weitestgehend farblos und geruchsneutral ist und darüber naturkosmetik-konform gewonnen werden kann.

Die Lösung dieser Aufgabe besteht in der
Verwendung von Triethylcitrat als Vergällungsmittel für Ethanol.

Vorteilhaft wird die Erfindung verwirklicht, wenn Triethylcitrat in Konzentrationen von mindestens 2,5kg, bevorzugt mindestens 5 kg, insbesondere mindestens 5,2 kg auf 100L reines Ethanol zugegeben.

Der Geschmack des Alkohols bzw. Zubereitungen, die diesen Alkohol enthalten, wird derart verändert, dass diese ungenießbar werden.

Dabei können Kosmetikprodukte bis zu 96 Gew% von diesen vergällten Ethanol enthalten. Triethylcitrat (Zitronensäuretriethylester, CAS-Nummer: 77-93-0) ist z.B. in der geforderten Qualität als Citrofol Al® oder Dermofeel TEC eco® erhältlich.

Zwar beschreibt die EP 0876811 eine Kombination von Stoffen als Vergällungsmittel für Parfüms und Kosmetika, wobei Triethylcitrat ein Bestandteil darstellt. Die Tatsache, dass Triethylcitrat als Einzelstoff ausreichend für die Vergällung sein kann wird daraus nicht deutlich.

Die naturkosmetik-konforme Herstellung von Triethylcitrat erfolgt über die Veresterung von Citronensäure mit Ethanol, wobei beide Ausgangsrohstoffe fermentativ gewonnen werden und frei von gentechnisch veränderten Organismen sind.

Struktur von Triethylcitrat

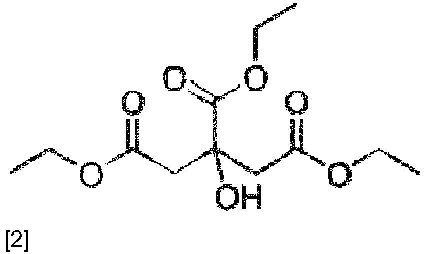

Triethylcitrat wird im Bereich der Kosmetikindustrie als Deo-Wirkstoff und Lösungsmittel u.a. für UV-Filter oder Parfümöle eingesetzt. [3] Weiterhin ist Triethylcitrat als besonders umweltschonender Weichmacher bekannt, der in diesen Bereich die stark persistenten Phthalate substituiert. Selbst in der Lebensmittelindustrie wird Triethylcitrat unter der E-Nummer 1505 als Lösungsmittel für Eiklarpulver und Aromen und gilt allgemein als gesundheitlich unbedenklich [4], [5]. Daher ist es umso überraschender, dass Triethylcitrat als Vergällungsmittel eingesetzt werden kann.

Emulsionen sind vorteilhafte Darreichungsformen im Sinne der vorliegenden Erfindung, z.B. in Form einer Creme, einer Lotion, einer kosmetischen Milch sind vorteilhaft und enthalten z.B. Fette, Öle, Wachse und/oder andere Fettkörper, sowie Wasser und einen oder mehrere Emulgatoren, wie sie üblicherweise für einen solchen Typ der Formulierung verwendet werden.

Medizinische topische Zusammensetzungen im Sinne der vorliegenden Erfindung enthalten in der Regel ein oder mehrere Medikamente in wirksamer Konzentration. Der Einfachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittel- und Arzneimittelgesetz).

Sofern die kosmetische oder dermatologische Zubereitung im Sinne der vorliegenden Erfindung eine Lösung oder Emulsion oder Dispersion darstellt, können als Lösungsmittel verwendet werden:
- Wasser oder wäßrige Lösungen
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder - monobutylether, Diethylenglykolmonomethyl- oder -monoethylether und analoge Produkte.

Insbesondere werden Gemische der vorstehend genannten Lösungsmittel verwendet. Bei alkoholischen Lösungsmitteln kann Wasser ein weiterer Bestandteil sein.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr.

Die wäßrige Phase der erfindungsgemäßen Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Erfindungsgemäß verwendete Gele enthalten üblicherweise Alkohole niedriger C-Zahl, z.B. Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wäßrig-alkoholischen oder alkoholischen Gelen vorzugweise ein Polyacrylat ist.

Feste Stifte enthalten z.B. natürliche oder synthetische Wachse, Fettalkohole oder Fettsäureester.

Übliche Grundstoffe, welche für die Verwendung als kosmetische Stifte im Sinne der vorliegenden Erfindung geeignet sind, sind flüssige Öle (z.B. Paraffinöle, Ricinusöl, Isopropylmyristat), halbfeste Bestandteile (z.B. Vaseline, Lanolin), feste Bestandteile (z.B. Bienenwachs, Ceresin und Mikrokristalline Wachse bzw. Ozokerit) sowie hochschmelzende Wachse (z.B. Carnaubawachs, Candelillawachs)

Als Treibmittel für aus Aerosolbehältern versprühbare kosmetische und/oder dermatologische Zubereitungen im Sinne der vorliegenden Erfindung sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Die nachfolgenden Beispiele sollen die vorliegende Erfindung verdeutlichen.

### Waschgel

| **Inhaltsstoff** | **Anteil in %** |
|---|---|
| Glycerin | 0,05 |
| Alkohol vergällt mit Triethylcitrat | 3 |
| Natriumbenzoat | 0,45 |
| Xanthangummi | 1,5 |
| Coco-Glucosid | 7,02 |
| Parfüm | 0,1 |
| Wasser | ad 100 |

### Gesichtswasser

| **Inhaltsstoff** | **Anteil in %** |
|---|---|
| Glycerin | 2 |
| Milchsäure | 0,09 |
| Natriumlaktat | 0,5 |
| Alkohol vergällt mit Triethylcitrat | 10 |
| Coco-Glucosid | 0,52 |
| Lävulinsäure | 0,2 |
| Natriumlävulinat | 0,1 |
| Parfüm | 0,07 |
| Wasser | ad 100 |

### O/W Lotion

| **Inhaltsstoff** | **Anteil in %** |
|---|---|
| Glycerin | 8,5 |
| Glycerylstearat | 2 |
| Cetylstearylalkohol | 2 |
| Alkohol vergällt mit Triethylcitrat | 7 |
| Dicaprylylether | 4 |
| Xanthangummi | 0,2 |
| Lävulinsäure | 0,2 |
| Natrium Lävulinat | 0,1 |
| Glycerylcaprylat | 0,3 |
| Rapsöl | 3,5 |
| Natriumstearoylglutamat | 0,2 |
| Parfüm | 0,1 |
| Wasser | ad 100 |

### O/W Creme

| **Inhaltsstoff** | **Anteil in %** |
|---|---|
| Glycerin | 8 |
| Glycerylstearat | 4 |
| Cetylstearylalkohol | 2 |
| Alkohol vergällt mit Triethylcitrat | 5 |
| Sheabutter | 1,5 |
| Dicaprylylether | 3 |
| Lävulinsäure | 0,2 |
| Natriumlävulinat | 0,1 |
| Rapsöl | 3,8 |
| Natriumstearoylglutamat | 0,3 |
| Anissäure | 0,2 |
| Parfüm | 0,1 |
| Wasser | ad 100 |

### O/W Creme

| **Inhaltsstoff** | **Anteil in %** |
|---|---|
| Glycerylstearat | 2 |
| Cetylstearylalkohol | 2 |
| Alkohol vergällt mit Triethylcitrat | 3 |
| Xanthangummi | 0,2 |
| Glycerylcaprylat | 0,3 |
| Rapsöl | 12 |
| Sheabutter | 2 |
| Hydrierte Kokosglyceride | 1 |
| Dehyracetsäure | 0,3 |
| Myristylmyristat | 1 |
| Glycerylstearatcitrat | 2,5 |
| Neutralöl | 4 |
| Parfüm | 0,1 |
| Wasser | ad 100 |

## Patentansprüche

1. Verwendung von Triethylcitrat als Vergällungsmittel für Ethanol.

2. Verwendung nach Anspruch 1, **dadurch gekennzeichnet, daß** Triethylcitrat in Konzentrationen von mindestens 2,5kg, bevorzugt mindestens 5 kg, insbesondere mindestens 5,2 kg auf 100L reines Ethanol vorliegen..

3. Kosmetische oder dermatologische Zubereitungen, enthaltend mit Triethylcitrat vergälltes Ethanol.
